# EUROPÄISCHE PATENTANMELDUNG

(11) **EP 0 525 353 A1**
(43) Veröffentlichungstag der Anmeldung: **03.02.1993**
(21) Anmeldenummer: 92110056.6
(22) Anmeldetag: 15.06.1992
(51) Int. Cl.: A61N 1/16, A61N 1/14

(54) **Ableitkabel für Umweltstörungen**

(30) Priorität: 17.06.1991 DE 4119899
(71) Anmelder: Ludwig, Wolfgang, Dr., D-72160 Horb (DE)
(72) Erfinder: Ludwig, Wolfgang, Dr., D-72160 Horb (DE)
(74) Vertreter: EGLI-EUROPEAN PATENT ATTORNEYS

(57) **Zusammenfassung**

Ein elektrisch leitendes kurzes Ableitkabel (6), vorzugsweise für geopathische "Aufladungen" von Metalleinlagen (2) in Unterbetten (1), ist elektrisch leitend mit der Metalleinlage (2) verbunden und mit einer Ferritdrossel (4) nahe der Verbindungsstelle mit der Metalleinlage (2) versehen. Die Metalleinlage (2) ist mit Magnetbändern (3) kombiniert und in diesen Magnetbändern (3) sowie in dar Ferritdrossel (4) sind in gleicher Weise physiologisch positiv wirksame Signale einprogrammiert (durch erzwungene Platzwechsel in der Gitterstruktur).

## Beschreibung

Die Erfindung betrifft ein Kabel aus elektrisch leitfähigem Material, das die von Metallen aufgefangene Umweltstörung vermindern oder beseitigen soll. In der Europäischen Patentanmeldung 91 103 017.9 vom 28.02.1991 werden Anordnungen beschrieben, die Umwelteinflüsse, z.B. geopathische Störungen mildern sollen, wie sie beispielsweise von dem Wiener Dozenten Dr.O.Bergsmann beschrieben wurden (1) Die in der genannten Europäischen Patentanmeldungen beschriebenen Anordnungen können nicht gegen elektrische Störfelder Abhilfe schaffen. Dagegen sind Metallmatten z.B. Kupfermatten bekannt, worüber z.B. das im Auszug in der Anlage beigefügte Gutachten des bekannten Schlafforschers Dr.U.J.Jovanovic vorliegt.

Bekannt ist es, daß eine Erdung solcher Kupfermatten, z.B. als Einlage in Schlafstellen, einen Potentialausgleich schafft, im Falle der heutigen Senderdichte aber auch die Bodenwellen von Sendern in die Kupfermatte hineinleitet, was erfahrungsgemäß erneut zu Schlafstörungen führen kann. Von der Baubiologie ist weiter bekannt, daß eine Erdung auch aus anderen Gründen nicht in allen Fällen biologisch sinnvoll ist (2) und daß sich andererseits Metalle über geopathischen Plätzen "aufladen". Eine computergeprüfte Theorie des französischen theoretischen Physikers J.E. Charon (3) erklärt die von Baubiologen gemachte Erfahrung, daß es sich bei solchen "Aufladungen" nicht nur um triviale elektrische Ladungen handelt.

Bekannt ist schließlich die Verwendung von Magnetbändern in Unterbetten zum Ausgleich magnetischer Anomalien, welche die Gesundheit beeinträchtigen können (4).

Die vorliegende Erfindung geht von den im folgenden beschriebenen Messungen und Personenversuchen aus und stellt sich die Aufgabe, nachteilige "Aufladungen" von Metallen - insbesondere im Schlafbereich - auf technisch einfache Weise für den Benutzer weitgehend unschädlich zu machen.

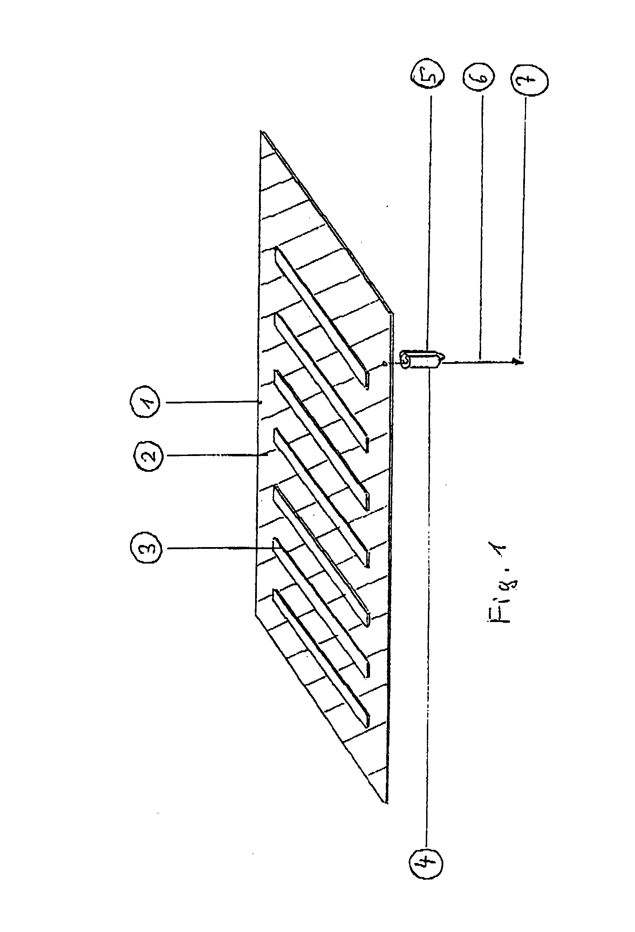

Erfindungsgemäß wird nach dem in Fig. 1 gezeigten Bespiel vorgeschlagen, anstelle eines langen Erdkabels ein kurzes frei in den Raum hängendes elektrisch leitfähiges Ableitkabel (6) mit einer Spitze (7) am Ende an die Metalleinlage (2) (beispielsewise in einem Unterbett (1) ) anzuschließen und daß zur Vermeidung einer Antennenwirkung eine Ferritdrossel (4) so angebracht wird, daß das Ableitkabel (6) zweimal durch die hohle Ferritdrossel (4) gezogen wird, wobei es außerhalb der Bohrung der Ferritdrossel (4) eine Schlaufe (5) bildet. Zusammen mit der Erdkapazität der Metalleinlage (2) wirkt dies als L-C-Siebkette.

Weiter wird vorgeschlagen, die bekannten Magneteinlagen (3) mit der Metalleinlage (2) zu kombinieren und die in der Europäischen Patentanmeldung 91 103 017.9 genannte Strukturmodifikation sowohl bei den Magnetstreifen als auch bei der Ferritdrossel (4) anzuwenden. Dadurch entsteht eine Resonanz zwischen den Magneteinlagen (2) und der Ferritdrossel (4), die "harmonische Schwingung" genannt wird (5), da sie eine physiologische meßbare positive Wirkung auf den Organismus ausübt, Die geopathischen Störungen repräsentieren demgegenüber "disharmonische Schwingungen" und sind in der "Aufladung" der Metalleinlage (2) gespeichert (entsprechend der Theorie von J.E.Charon,3). Die mit harmonischen Rhythmen programmierte Ferritdrossel (4) wirkt für diese wie ein Sperrkreis, d.h. leitet sie nicht in die Umgebung ab (wobei es sich elektrisch um eine Rekombination negativer mit positiver Ladungen handelt). Pathologische ("disharmonische") Informationen können jedoch über das Ableitkabel (6) das Unterbett (1) verlassen, da sie nicht in Resonanz mit den Schwingungen der Ferritdrossel (4) kommen. Die kristallinen Resonatoren in der Ferritdrossel (4) werden durch die Resonanz mit den ebenso programmierten Magnetbändern (3) angeregt und schwingen dadurch in höheren Energieniveaus, was die positive Wirkung der Ableitkabel (6) verstärkt.

Diese zunächst ungewöhnlich erscheinende Erklärung läßt sich objektiv prüfen, wobei Arbeiten an der Universität Mailand (6) zum Tragen kommen: Stellt man auf eine Störstelle mehrere Stunden lang eine Wasserprobe, so verändert sich diese auch nach dem Wegnehmen von der Störstelle in den nächsten Tagen charakteristisch. Dies läßt sich sowohl mit einem Niederfrequenz-Spektrometer nach Fig. 2 als auch mit einem Zweistrahl-SpektralPhotometer im Ultraviolett-Bereich messen (Fig. 3 bis 5). Die dabei benutzte Wasserprobe war im vorliegenden Fall mit Umkehrosmose gefiltertes Leitungswasser aus dem Schwarzwald. In Fig. 5 ist die Prüfanordnung eingezeichnet:

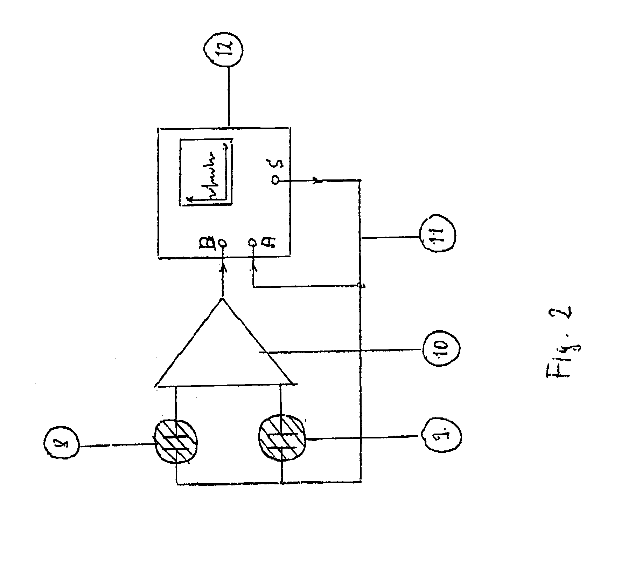

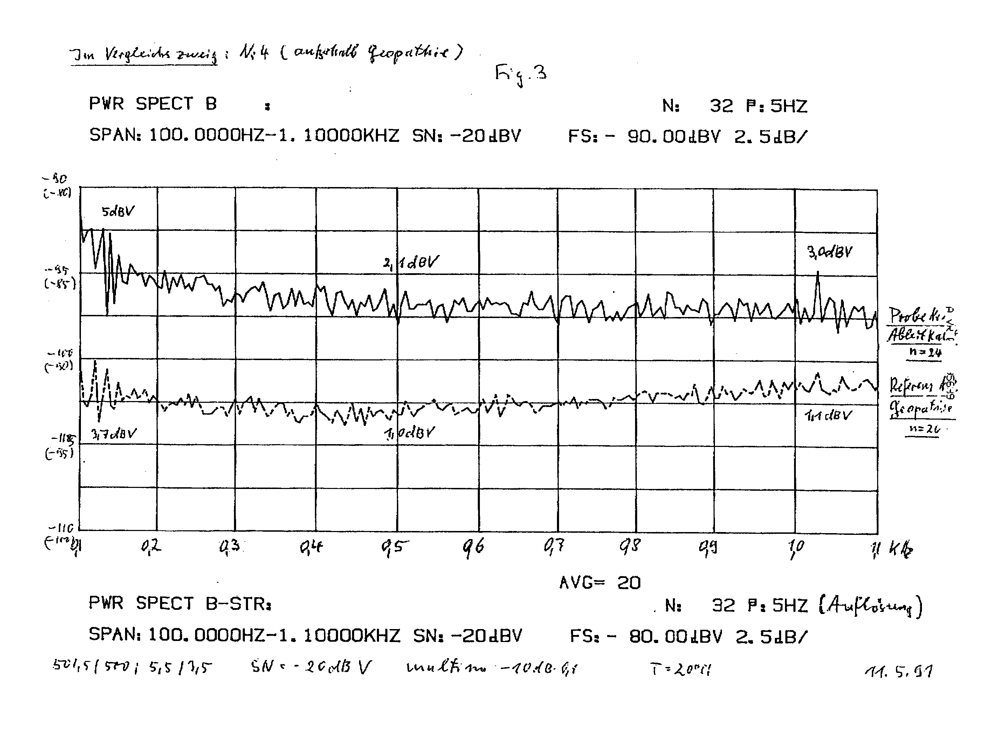

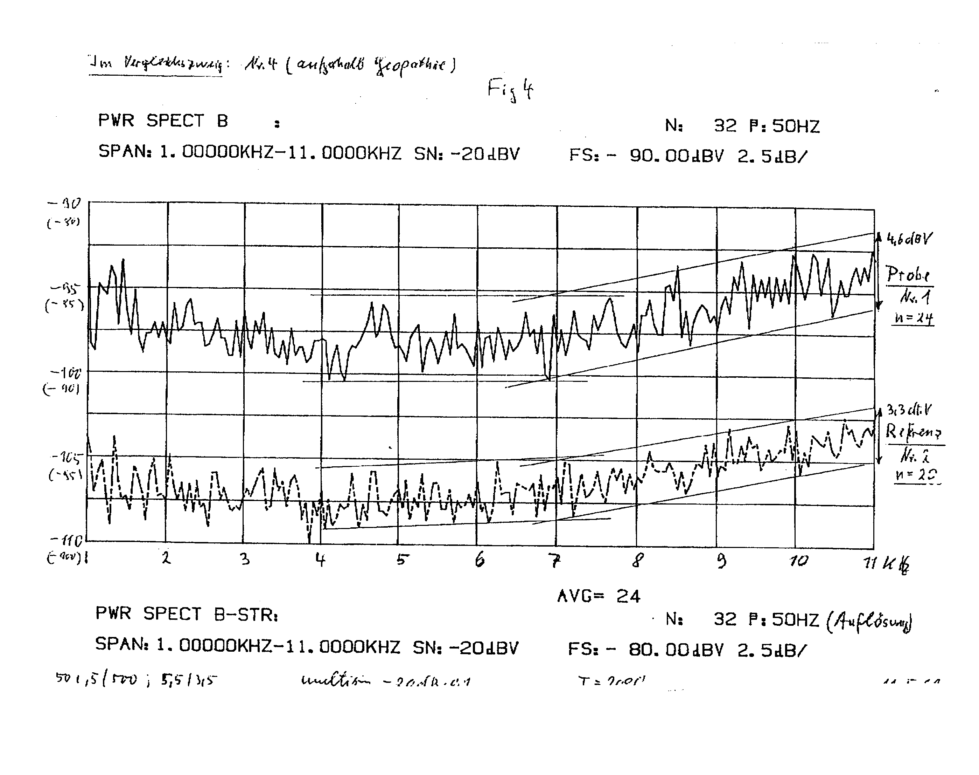

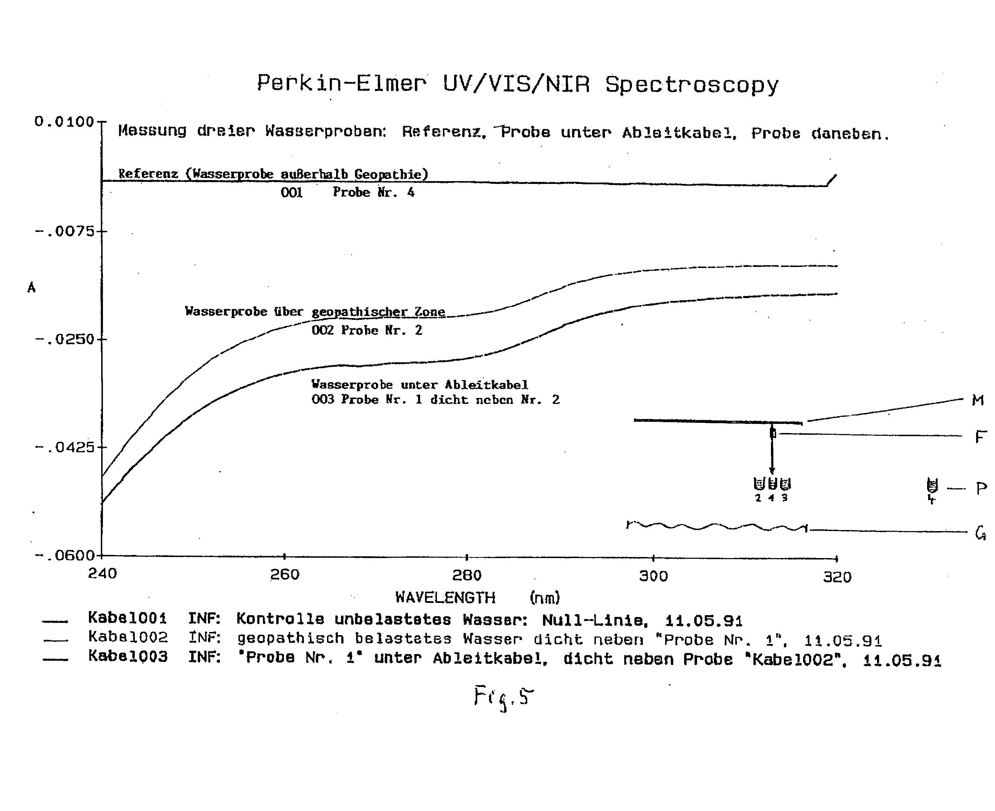

Das Material M (bestehend aus dem Unterbett (1) mit Metalleinlage (2) und Magnetstreifen (3) wird über einer geopathischen Störstelle G aufgestellt. Solche Plätze weisen eine signifikant erhöhte Gammastrahlung aus dem Boden auf (natürliche Radioaktivität, im Beispiel 2,3mal so hohe Zählrate als in der weiteren Umgebung, gemessen mit einem Geigerzähler). Das Ableitkabel (6) mit der Ferritdrossel F (in Fig. 1 mit (4) bezeichnet) endet mit seiner Spitze (7) dicht über einem Gefäß mit Wasser (Nr. 1 in Fig. 5). Dicht daneben stehen Referenzgefäße mit dem gleichen Wasser (Nr. 2 und 3 in Fig.5) und auf neutralem Platz (mit normaler Gammadosis) in der Nähe eine vierte Probe (Nr. 4 in Fig. 5). G in Fig. 5 markiert die geopathische Zone (im Beispiel ca. 80 cm x 80 cm).

Gemäß Fig. 2 wurden die Spektren Fig. 3 und 4 aufgenommen. In Fig. 2 bedeuten: (8) die Probenküvette mit zwei Goldelektroden, (9) die Referenzküvette, (10) ein nach Betrag und Phase abgeglichener Differenzverstärker, (11) das Zuführungskabel eines "Multisinus-Signals", das aus dem Signal-Analysator (12) mit Plotter kommt und synchron mit den Fourierfiltern im angegebenen Frequenzbereich (0,1) bis 1,1 kHz bzw. 1 bis 11 kHz) automatisch mehrmals durchläuft (20- bzw. 24ma! zur Ausmittelung des Geräterauschens). In die Referenzküvette (9) wird die Wasserprobe Nr. 4 (Fig. 5) gegeben. Die strichlierte Kurve (jeweils untere Kurven in Fig. 3 und 4) repräsentiert die Probe Nr. 2 (Fig. 5) bzw. Nr. 3 in der Probenküvette (8). Die vom Wasser aufgenommenen Störsignale liegen zwischen 1 und 3,3 Dezibel-Volt (dBV). Die ausgezogenen oberen Kurven in Fig. 3 und 4 repräsentieren die Probe Nr. 1 in der Probenküvette (8) und liegen in ihren Störsignalen wesentlich höher (2,1 bis 5 dBV): In der Nähe von 100 Hz beträgt der Unterschied 1,35 dBV; bei ca. 500 Hz 2,1 dBV; bei ca. 1 kHz 2,7 dBV und bei ca. 10 kHz 1,4 dBV (Meßgerät: Rockland Signal Analyzer 5830 B).

Fig. 5 zeigt die Messung im Ultraviolett-Bereich drei Tage nach Exposition, aufgenommen mit einem Perkin-Elmer Lambda 2-Spektralphotometer. Fünf Tage nach Exposition ergab sich das gleiche Bild (wobei anstelle Probe Nr. 2 Probe Nr. 3 verwendet wurde). Im Vergleichsstrahl war wieder die unbelastete Wasserprobe (Nr. 4 in Fig. 5). Die Küvetten waren fünf cm lange Quarzküvetten (unterhalb von 240 nm absorbiert das verwendete Wasser sehr stark, sodaß unter 240 nm nicht mehr registriert werden konnte). Die obere gerade Linie zeigt die Messung der gleichen Probe Nr. 4 in der Probenküvette. Dazu wurde zunächst mit 2 x Probe Nr. 4 ein Nullabgleich durchgeführt ("background correction") und dann nochmals Nr. 4 in der Probenküvette ausgetauscht, um den Einfluß des Umfüllens zu kontrollieren. Es ergaben sich keine meßbaren Abweichungen von der Nullkorrektur. Beide Proben Nr. 4 unterscheiden sich logischerweise nicht.

Die mittlere Kurve zeigt Probe Nr. 2 (immer gegen Nr. 4 im Vergleichsstrahl) und die untere Kurve Probe Nr. 1. Beide Proben Nr. 1 und 2 haben sich durch die geopathische Störung stark verändert und sind gegenüber UV transparenter geworden (negativer Extinktions-Koeffizient A in Fig. 5): Aufbrechen von Wasserstoffbrücken in den resonanzgekoppelten Clustern (6). Die Expositionszeit der Proben Nr. 1 bis 4 betrug 24 Stunden. Die Probe Nr. 1 hat - wie auch in Fig.3 und 4 zu sehen - noch mehr abbekommen, nämlich die von der relativ großen Materialfläche M (Unterbett (1) mit Metalleinlage (2) und Magneteinlage (3) ) aufgefangene und über das Ableitkabel (6) in die Wasserprobe Nr. 1 abgeleitete Störung. Die Wasserproben Nr. 1 bis 4 hatten je eine Grundfläche von 3 cm². Damit ist objektiv gezeigt, daß das Ableitkabel (6) tatsächlich "disharmonische Signale" aus dem Unterbett (1) mit Metalleinlage (2) und Magnetbändern (3) ableitet, seien es nun - nach Chanon - Elektronen mit physiologisch negativer Information oder andere Phänomene, die noch nicht erforscht sind (es werden u.a. Axionen diskutiert).

Die Strukturmodifikation von Magnetbändern (3) und Ferritkern (4) läßt sich anhand solcher Kontrollmessungen optimieren, wie es in der Europäischen Patentanmeldung 91 103 017.9 beschrieben ist. Versuchspersonen haben die Anordnung nach Fig' 1 getestet und im Blindversuch ergab sich, daß ohne Ableitkabel (6) über geopathischen Störzonen Schlafstörungen auftraten, mit Ableitkabel (6) jedoch nicht mehr.

Vorteile der Erfindung sind die technisch einfache Ausführung des Ableitkabels zur Verminderung oder gar Beseitigung geopathisch bedingter "Aufladungen" von Metallteilen, z.B. Metalleinlagen (2) in Unterbetten (1), die Verbesserung dieser Wirkung durch gleiche Strukturmodifikation einer Ferritdrossel (4) und von Magnetbändern (3) gemäß eines Verfahrens nach der Europäischen Patentanmeldung 91 103 017.9, die Kombination von Metalleinlage (2) und Magentbändern (3) mit Ableitkabel (6) und Ferritdrossel (4) und der Wegfall eines langen Erdkabels bis zur nächst-möglichen Erdverbindung (die ungestört sein muß), sowie die Möglichkeit, die Strukturmodifikation durch Kontrollmessungen im NF- und UV-Gebiet zu optimieren , ausgenützt wird.

Gegenstände der Erfindung sind ein mit der Metalleinlage (2) (z.B. im Unterbett (1) ) elektrisch leitend verbundenes Ableitkabel (6) kurzer Länge (das einige cm über dem Fußboden mit einer Spitze (7) endet), eine Ferritdrossel (4) in dem Ableitkabel (6) dicht an der Verbindungsstelle zwischen Ableitkabel (6) und Metall-Einlage (2), die Kombination von Metalleinlage (2) mit bekannten Magnetbändern (3) sowie die gleiche Strukturmodifikation von Magentbändern (3) und Ferritdrossel (4). Gegenstand der Erfindung ist weiter die Optimierung der Strukturmodifikation anhand von Kontrollmessungen an Wasserproben.

Die Erfindung wird anhand eines in der Zeichnung Fig. 1 dargestellten Ausführungs-Beispiel näher erläutert. Ein objektiver Beweis der Wirkung wird anhand der Fig. 2 bis 5 beschrieben.

Es zeigen:
Figur 1 ein Unterbett (1) mit der Metalleinlage (2) und den Magnetbändern (3). Die Metalleinlage (2) ist elektrisch leitend verbunden mit dem Ableitkabel (6), das eine Ferritdrossel (5) und eine Spitze (7) enthält.
Figur 2 eine Meßanordnung für Wasserproben im Niederfrequenz-Bereich. Die Probenküvette (8) mit zwei Goldelektroden und die Referenzküvette (9) sind an einen nach Betrag und Phase abgeglichenen Differenz-Verstärker (10) angeschlossen. Das Differenzsignal wird an den Eingang B des Signal Analysators (Fourier-Analysator) (12) gegeben, der ein synchron mit dem Fourierfilter durchlaufendes Sinus-Signal S produziert. Dieses wird an den Eingang A des Signal-Analysators (12) über die Verbindung (11) gegeben und außerdem an die Probe- und Referenz-Küvetten (8) und (9), deren Transferfunktion in Differenz gemessen wird (der Signal Analysator (12) gestattet auch Nyquist-Diagramme darzustellen, wozu der Eingang A notwendig wird).
Figur 3 ein Meßergebnis im Frequenzbereich von 0,1 bis 1,1 kHz, wobei die Ordinate im lorarithmischen Maßstab in Dezibel-Volt geeicht ist. SN = sensitivity = Grundempfindlichkeit; FS = full scale = Vollausschlag = obere Ordinatengrenze (für die ausgezogene obere Probenkurve = -90 dBV, für die strichlierte untere Referenzkurve = -80 dBV, absichtlich versetzt zur Unterscheidung); 2.5 dB/ = 2,5 dB pro Ordinaten-Teilstrich; N = vorgewählte Wiederholungsrate; AVG = wirklich benutzte Wiederholungsrate (AVG = average). Absichtlich wurde bei der Probe Nr. 1 bis AVG = 24 und bei dem Referenzwasser bis AVG = 20 aufgenommen, d.h. die Probe Nr. 1 besser gemittelt als die Probe Nr. 2 (Referenz), sodaß die großen Signale bei Probe Nr. 1 nicht etwa einem größeren Rauschen zugeschrieben werden können. β:5 Hz = Auflösung. Das Ableitkabel (4) hat erheblich mehr Störungen in die Wasserprobe Nr. 1 abgeleitet als die Referentprobe Nr. 2 durch die Geopathie aufgenommen hat.
Figur 4 analog Fig. 3 im Frequenzbereich 1 bis 11 kHz. Hier ist die Auflösung zehnmal schlechter (50 Hz), weshalb die Signale bei 1 kHz gegenüber Fig. 3 aufsummiert und z.T. gemittelt erscheinen. Im übrigen gilt das zu Fig. 3 gesagte.
Figur 5 ein Meßergebnis im UV-Gebiet zwischen 240 und 320 Nanometer. Auf der Ordinate ist der Extinktionskoeffizient A der Differenz zwischen Probe Nr. 1 (untere Kurve), Nr. 2 (mittlere Kurve), Nr. 4 (obere Gerade) und jeweils Nr. 4 (im Vergleichsstrahl) aufgetragen (in den USA wird der Extinktionskoeffizient "absorbtion coefficient" genannt, daher die Abkürzung A). In der Skizze rechts in Fig. 5 bedeuten: M = Metalleinlage (2); F = Ferritdrossel (4); P = Proben (Nr. 1 bis 4); G = gₑₒpₐtₕische Zone. In Betracht gezogene Druckschriften:
   (1) O. Bergsmann: Risikofaktor Standort. Facultas-Universitätsverlag Wien 1990
   (2) H. Palm: Das Gesunde Haus. Ordo-Verlag Konstanz, 5. Auflage 1985
   (3) J.E. Charon: Theorie de la Relativite Complexe, Ed.Albin Michel-Verlag Paris 1977
   (4) V.E.Archer: Geomagnetism,Cancer, Weather and Cosmic Radiation. Health Physics Pergamon Press 34, 237-247, 1978
   (5) H. Wolf: Harmonische Schwingungen. Verlag Natürlich und Gesund Stuttgart 1990
   (6) E. del Giudice, G.Preparata and G. Vitiello: Water as a free Electric Dipole Laser. Physical Rev. Let. 61, 1085-1088,1988

## Patentansprüche

1. Ableitkabel für Umweltstörungen, dadurch gekennzeichnet, daß das Ableitkabel (6) elektrisch leitend mit dem die Umweltstörungen auffangenden Metall (2) verbunden ist und in Nähe der Verbindungsstelle eine strukturmodifizierte Ferritdrossel (4) enthält.

2. Ableitkabel nach 1. Anspruch, dadurch gekennzeichnet, daß die gleiche Strukturmodifikation bei Magnetstreifen (3) verwendet wird, die sich in Nähe des die Umweltstörungen auffangenden Metalls (2) befinden.

3. Ableitkabel nach einem der vorhergehenden Ansprüche, dadurch gekennzeichnet, daß die Wirkung der Ableitung spektroskopisch an Wasserproben geprüft und die Strukturmodifikation danach optimiert wird.
